# EUROPEAN PATENT APPLICATION

(11) **EP 4 250 311 A1**
(43) Date of publication of application: **27.09.2023**
(21) Application number: 22166954.2
(22) Date of filing: 06.04.2022
(51) Int. Cl.: G16H 40/40, G06F 9/451

(54) **ULTRASOUND SYSTEM WITH CUSTOMIZATION UNIT**

(30) Priority: 24.03.2022 US 202263323101 P
(71) Applicant: Koninklijke Philips N.V., 5656 AG Eindhoven (NL)
(72) Inventor: BHARAT, Shyam, Eindhoven (NL); BULUT, Murtaza, Eindhoven (NL); BERA, Deep, Eindhoven (NL); SUTTON, Jonathan Thomas, Eindhoven (NL); HIWALE, Sujitkumar, Eindhoven (NL); VAN LIESHOUT, Ron Martinus Laurentius, Eindhoven (NL)
(74) Representative: Philips Intellectual Property & Standards

(57) **Abstract**

The invention relates to an ultrasound system (200) operable by one or more users of the ultrasound system to process ultrasound measurements of a patient. The system comprises an ultrasound unit operable to perform the ultrasound measurements of the patient. The system further comprises a customization unit. The customization unit obtains user interaction data of one or more interactions of a current user performing a current task at the ultrasound system. The ultrasound system provides user-facing functionality for performing the current task at multiple different skill levels. The customization unit determines, from the user interaction data, a real-time skill estimate of the current user. The real-time skill estimate indicates a current ability of the current user to perform the current task. The customization unit customizes the ultrasound system for performing the current task at a skill level corresponding to the real-time skill estimate of the current user.

## Description

### FIELD OF THE INVENTION

The invention relates to an ultrasound system, a customization unit for use in the ultrasound system, and to a computer-implemented method corresponding to the customization unit. The invention further relates to a computer-readable medium.

### BACKGROUND OF THE INVENTION

Currently, ultrasound is often used in the intensive care unit (ICU) as a standalone tool to perform spot checks in patients and for specific tasks such as line placement. When a scan is desired, the machine is often brought in by the intensivist, or an order is placed out to the cardiology department or to other relevant ultrasound scanning department. Typically, the results stay in the ultrasound ecosystem, and are reviewed there. Therefore, most of current ultrasound use in the intensive care unit is by experts.

However, as invasive tools to monitor patient hemodynamics fall out of favour, ultrasound is being increasingly used as a monitoring tool in critical care, perioperative environments. Due to this change in use, ultrasound in the ICU and other hospital units is likely to be performed more and more by users of different clinical roles, e.g., lead intensivist, junior clinician, resident, nurse etc.; with different skill levels, and with varying time constraints. Users of different roles may have different levels of training and their goal for US use may also be different. Moreover, the level of training with use of ultrasound varies significantly with clinical specialty and institutional preferences.

Another development is that ultrasound is being used more and more in non-hospital settings, such as at home, in an ambulance, or at a sports facility. Also in this case, ultrasound may have a wide range of users with different skills, goals, and time constraints.

### SUMMARY OF THE INVENTION

It would be desirable to provide ultrasound systems that can be effectively used by a wide range of users.

In accordance with a first aspect of the invention, an ultrasound system is provided, as defined by claim 1. In accordance with further aspects of the invention, a customization unit for use in the ultrasound system is provided, as defined by claim 13; and a computer-implemented method corresponding to the customization unit is provided, as defined by claim 14. In accordance with an aspect of the invention, a computer-readable medium is described as defined by claim 15.

Various measures discussed herein relate to an ultrasound system. The ultrasound system may be operable by one or more users of the ultrasound system to process ultrasound measurements of a patient. The processing may involve preparation of the ultrasound scanning; image acquisition; and/or analysis of the acquired ultrasound measurements. The processing may be performed at various units of the ultrasound system, for example: image acquisition may be performed at the ultrasound unit that performs the ultrasound measurements; analysis may be performed at a patient monitor that allows to inspect the ultrasound images or other types of measurements of the ultrasound unit; and scan preparation may be performed at the ultrasound unit and/or the patient monitor. The ultrasound system may comprise at least the ultrasound unit.

In order to improve the usability of the ultrasound system, the inventors envisaged to use a customization unit. The ultrasound system may provide user-facing functionality, e.g., a user interface, for performing one or more tasks at the ultrasound system. Interestingly, the functionality may be provided at multiple different skill levels that differ, for instance, in terms of the amount of user guidance and/or automation that is provided. By customizing the ultrasound system appropriately to provide the user-facing functionality at a skill level for the user at hand, the ultrasound system may be made more effectively usable by different users. It is noted that providing customizability typically does not just involve adapting a user interface per se, but also providing further components that enable the customization, e.g., software units to provide user guidance and/or automation at respective skill levels, and/or trained machine learning models, sensors, storage, etc., used to provide the user guidance or automation.

Interestingly, the customization may be based on a real-time skill estimate of a current user who is performing a current task at the ultrasound system. The customization unit may be configured to obtain user interaction data of one or more interactions of the current user. For example, the user interaction data may comprise one or more respective interaction items representing respective individual interactions of the user with the system, such as a button press, a selection of a scanning mode, and/or a selection of an image quality estimation method. An interaction item may also represent an aggregate quantity corresponding to a set of interactions, for example an amount of time and/or an amount of steps taken to complete a given task.

From the user interaction data, the customization unit may determine a real-time skill estimate of the current user. The ultrasound system may be customized by providing the user-facing functionality at a skill level corresponding to the determined real-time skill estimate. Interestingly, by basing the skill estimate on the user interaction data, a skill estimate may be obtained that indicates a current ability of the current user to perform the current task, e.g., indicative of both skill level and current physical and/or mental capability. In particular, the skill estimate may not be derived by simply identifying the user and/or user role and selecting a skill estimate for the identified user and/or role. In particular, in different circumstances, different skill estimates may be determined for the same user performing the same task, depending on the interaction data. For example, the same user may perform the same task a first time, wherein a first real-time skill estimate is determined, and a second time, wherein a second and different real-time skill estimate is determined. This may be despite the fact that there may have been no change in a user profile; in fact, the determination of the skill estimate may not be based on any identification of the current user. Instead, the skill estimate may be determined based on how the current user interacts with the system according to the user interaction data, and accordingly, the skill estimate may indicate the current ability of the user to perform the task and may in that sense be seen as real-time.

Using a real-time skill estimate, instead of for example looking up an appropriate skill level given the user's identity, has several advantages. As the inventors realized, the user's skills may be influenced by their current mental and physical state, and as such, can change over time. In particular, the user's skill level may be affected by the context in which the current task that is being carried out, e.g., by the urgency or stress of the situation, and/or by the level of cooperation provided by the patient. Also without taking into account context-dependent factors, it may be hard in practice to manually set an appropriate skill level for a user, for example, to derive the skill level from a paper questionnaire or similar. Determining the skill level in an automated way based on current interactions, avoids this difficulty. When manually setting the skill level, there is also the problem that user skills typically change with time, and it is hard and error-prone to keep the profile up to date to reflect the changed skills. For example, users may get better at what they do over time without themselves or others even being aware of this. Relying (solely) on a profile of an identified user is also error-prone in case several people are involved, in which case in practice often no reidentification takes place in case another user takes over; and it cannot be used to provide customization for new users that do not have a profile yet, both inside hospitals and outside.

By providing the user-facing functionality at a skill level appropriate for the context at hand, the user may be assisted to interact with the ultrasound system, such as the ultrasound unit or patient monitor, in a more efficient way. Taking into account such context, the user-facing functionality may be concentrated on the most important value-adds for that user. For example, workflow suggestions may be provided only on aspects relevant to the present context, thereby avoiding guidance overload. Moreover, by providing guidance at lower skill levels, errors by such users may be made less likely; and the time of examination and need to re-do examination may be minimized.

The user-facing functionality may not be adapted to the subjective preferences of any particular user; rather, by collecting user interaction data and using this user interaction data to estimate a skill level, for example without identifying the user at hand and thus independently of that user's subjective preferences, technical measures may be provided that allow to provide automation and/or guidance in the most appropriate way for the current situation. Thus, by providing customization in a data-driven way depending on the situation at hand, instead of based on user preferences, the efficiency of interacting with the ultrasound system as a whole, as opposed to only for some particular usage patterns and/or for some particular users, may be improved. For example, the number of user actions needed measured over many interactions of different users with different roles and/or in different situations, may be decreased.

The unit of the ultrasound system that provides the customization, e.g., that determines the real-time skill estimate and customized the ultrasound system based on it, may be referred to herein as a customization unit. The customization unit can generally take various forms. The customization unit may for example be comprised in the ultrasound unit, in a patient monitoring unit, a bridge unit, or any other unit of the ultrasound system. The customization unit may be part of the same device that provides the user-facing functionality for the current task, but also this is not needed, e.g., the customization device may customize the ultrasound system according to the real-time skill estimate by determining the skill level for the current task and providing an instruction to the device that provides the user-facing functionality, to provide this functionality at this skill level.

Optionally, the ultrasound system may be for use in a critical care and/or perioperative environment, for example, in an intensive care unit (ICU). Especially in such settings, ultrasound is expected to be used more and more by users of different clinical roles and skill levels, for example, to replace as invasive tools to monitor patient hemodynamics. Thus it is particularly relevant to provide appropriate guidance in this setting.

Optionally, the ultrasound system may be for use outside of a hospital, for example, at home, in an ambulance, or at a sports facility, or in a primary clinic. For example, the ultrasound system may be used in situations where stationary diagnostic systems may not be not available, e.g., in less developed counties or regions or rural areas. Also in such uses, ultrasound may be used by a wide range of users, in particular by relatively inexperienced users. Moreover, outside of a hospital it may be hard or infeasible to identify the user or to access a stored profile of the user, and there may be a big variability in terms of environmental factors (other people present, stressfulness of the situation, etc). so that instead using interaction data to determine a real-time skill estimates is particularly relevant. In such scenarios, the vitals or images measured on the spot (e.g., ultrasound images of sports injuries) and/or personal health data (e.g., reference ultrasound images) may be used for analysis.

Optionally, the customization unit may be configured to determine the real-time skill estimate independent from any user identification. For example, the customization unit may not obtain a user identifier of the user, or in any case, may not use any such user identifier when determining the real-time skill estimate. Accordingly, the customization may be performed to provide the user-facing functionality in the most efficient way for the situation at hand, regardless of any subjective user preferences that the particular user might have. This has several advantages. It allows to provide customization without the need to access user profile information, which makes the system easier to integrate into existing workflows where no identification currently takes place, in particular in non-hospital settings; while also improving security, since no access to a source of profile information is needed. Not performing user identification is also beneficial from a privacy point of view: users may feel uncomfortable with their skill levels being traced, but can still benefit from customization performed in an anonymized way. The use of data-driven customization also allows the customization to be optimized in a feedback loop, e.g., by collecting data on the effect of performing a certain customization based on certain interaction data; e.g., manually, or by training a machine learning model for determining the real-time skill estimate.

Optionally, the ultrasound system is an ultrasound for monitoring (US4M) system. Generally, the term US4M system may refer to a system in which the ultrasound unit is functionally integrated with a patient monitoring unit, for example, to integrate ultrasound images and/or measurements into the patient monitoring ecosystem. As known per se, such a patient monitor (sometimes also referred to as a "medical monitor", "physiological monitor", or "vital signs monitor") may be configured to display current measurements of one or more physiological parameters of the patient, e.g., blood pressure, pulse oximetry, and/or ECG. Instead of or in addition to such measurements, the patient monitor of the US4M system may be configured to show information based on the measurements of the ultrasound unit. For example, the patient monitor may be configured to show the ultrasound images themselves, or to show current values of physiological parameters derived from the ultrasound images such as an anatomic dimension, e.g., a volume of a heart chamber or of another body part. In such a US4M system, users may perform ultrasound tasks, such as scan preparation, acquisition, and analysis, at various units of the US4M system. In an embodiment, the current task may be performed at the patient monitoring unit. In a further embodiment, the current task may be performed at a bridge device that is used for information exchange between the ultrasound unit and the patient monitoring unit. Accordingly, the user-facing ultrasound-related functionality at these respective units of the ultrasound system may be customized as described herein.

Optionally, the customization unit may be further configured to derive from the user interaction data the current task that the current user is performing. This has the advantage that also this can be performed automatically without e.g. the need for the user to manually select the current task. However, the current task can also be derived by other means. An alternative way of deriving the current task is for example to use data captured from an expert guidance subsystem of the ultrasound system, for example a system for providing remote expert guidance based on video and/or audio.

Optionally, the user-facing functionality may be for performing the current task at multiple different skill levels and according to multiple different roles. For example, the current task may be performable according to a first role, and the ultrasound system may be configurable to provide user-facing functionality for the current task according to multiple different skill levels of the first role. And, the current task may be performable according to a second role, and the ultrasound system may be configurable to provide user-facing functionality for the current task according to multiple skill levels of the second role. The user interfaces for different role-skill level combinations may partially overlap, but are typically not completely the same. Accordingly, the customization unit may obtain the current role; determine the real-time skill estimate; and customize the ultrasound system according to the role and skill estimate. For example, the customization unit may be configured to customize the ultrasound system for at least one or two of the following roles: intensivist, lead intensivist, clinician, junior clinician, resident doctor, nurse, general practitioner, midwife, sports medic, home caregiver, and the patient themself.

Generally, the current task may be part of a current stage of multiple stages of the processing of the ultrasound measurements. For example, the current stage may be preparation; image acquisition; or analysis. Generally, analysis may involve interpretation of the ultrasound images or other measurements; curation (e.g., selection of images or other measurements for storage or further processing); and data transfer.

Optionally, the real-time skill estimate may be determined for the current stage from interactions of the current user only at the current stage. Accordingly, the real-time skill estimate may be specific to the current stage and may therefore be better adapted to the context of the current task. For practical purposes, in a given stage, the user or users interacting with the ultrasound system may be assumed to remain the same. Thus, using data from the current stage only may reduce pollution of the user interaction data with data of other users, and/or with data that is less relevant. This may be beneficial especially when using machine learning to determine the skill estimate. On the other hand, even when using interaction data only of the current stage, it is still possible to use a real-time skill estimate and/or role derived at an earlier stage as a cue, e.g., as an initial guess as to the current real-time skill estimate and/or role that may be confirmed or refuted as interaction data of the current stage is collected.

Generally, the user interaction data that is used to determine the real-time skill estimate, may comprise multiple interaction items. The interaction items may comprise one or more interaction items representing respective individual interactions of the user with the system, for example, individual inputs provided by the user and/or individual actions performed by the user as part of the current tasks. For example, the set of interaction items may comprise interaction items representing one or more of: a button press, a selection of a scanning mode, a selection of an image quality estimation method, an access of a prior image, a review of an imaging order, an access of prior image data, a measurement performed on an ultrasound image, a quantification performed on an ultrasound image, an image quality estimation method applied to an ultrasound image; an access of patient information relating to the patient being imaged; and an inspection and/or used viewing settings of one or more signals at a patient monitor. Instead of or in addition, the interaction items may comprise interaction items representing aggregate quantities determined over multiple interactions of the user with the system (e.g., computed by the customization unit itself, or received in computed form). For example, the set of interaction items may comprise interaction items representing one or more of: a time-to-obtain first image, a number of images saved, a time to achieve a target view, and a number and/or types of manual edits performed on an auto-quantification result.

Generally, the interaction items may be obtained in various ways, e.g., interactions with a user interface of the ultrasound system (such as with a keyboard, mouse, touch screen, etc.) may be logged by the device that provides the user interface or captured by a camera recording the user. At least one of the interaction items may be based on sensor measurements of the ultrasound system apart from the ultrasound measurements themselves. For example, one or more interaction items may be used that are based on sensors of the ultrasound probe and that indicate how the probe is used, for example, by indicating an area of the body that has been scanned; by indicating an inserted pressure and/or by representing a movement pattern of the ultrasound probe.

Optionally, the ultrasound unit may, in addition to the user interaction data, use context information about the current task when determining the real-time skill estimate. The context information may include information about the patient, such as an estimate, based on patient information apart from the ultrasound image itself, of a difficulty of imagining the patient in their current condition. Instead or in addition, the context information may include information about the physical environment in which the ultrasound system is used, such as a number of people present in the room where the ultrasound system is used, measurements of air quality and/or noise, and the like. Such aspects can influence physical and psychological state of the user, which can interact with the real-time skill levels.

Generally, the customization unit may customize the ultrasound system to operate at a given skill level in various ways. One way is to provide, or not to provide, a user guidance depending on the skill level, for example whether or not to show an acquisition guidance module for acquiring a particular type of image, such as an A4C image (which module may be as known per se). Providing such guidance to users with less skills may allow them to work faster and make less mistakes, while such guidance may only serve to distract a user with a higher skill level. Another way of customizing, is to automate or not to automate a certain operation, for example, to automatically pre-load a predefined set of scan settings. Also here, automating may prevent less experienced users from making mistakes and may allow them to work faster, while allowing more experienced users to achieve a higher degree of quality by being able to customize the operation, and not distracting them.

As a particular example, the customization unit may be configured to customize the ultrasound system by, depending on the skill level, automatically retrieving and showing, or not automatically retrieving and showing, information about a prior scan for a current follow-up scan. As another example, the customization unit may be configured to customize the ultrasound system by, depending on the skill level, automatically pre-loading, or not automatically pre-loading scan settings suitable for the current task (e.g. line placement). In yet another example, the current task may relate to obtaining a given view of an organ. The customization unit may in this case be configured to customize the ultrasound system by, depending on the skill level, showing or not showing example images of the given view of the organ.

Optionally, the customization unit may allow the user to learn and/or modify the skill level at which the ultrasound system offers the current task to the user. In other words, the user may have a manual override, by which they can see and correct the customization that is performed, as desired. Providing such a manual override makes it possible to use the automated customization even if it does not always derive the right real-time skill estimate. The activation of the manual override may be logged, e.g., in combination with the used user interaction data, such that the determined real-time skill estimate can be reviewed, e.g., manually or automatically by re-training.

It will be appreciated by those skilled in the art that two or more of the above-mentioned embodiments, implementations, and/or optional aspects of the invention may be combined in any way deemed useful.

Modifications and variations of any computer-implemented method and/or any computer readable medium, which correspond to the described modifications and variations of a corresponding system, device, or unit, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a customization unit for use in an ultrasound system;
Fig. 2 shows an ultrasound system;
Fig. 3. shows a detailed example of how to customize an ultrasound system;
Fig. 4 shows a computer-implemented method of customizing an ultrasound system;
Fig. 5 shows a computer-readable medium comprising data.

It should be noted that the figures are purely diagrammatic and not drawn to scale. In the figures, elements which correspond to elements already described may have the same reference numerals.

### DETAILED DESCRIPTION OF EMBODIMENTS

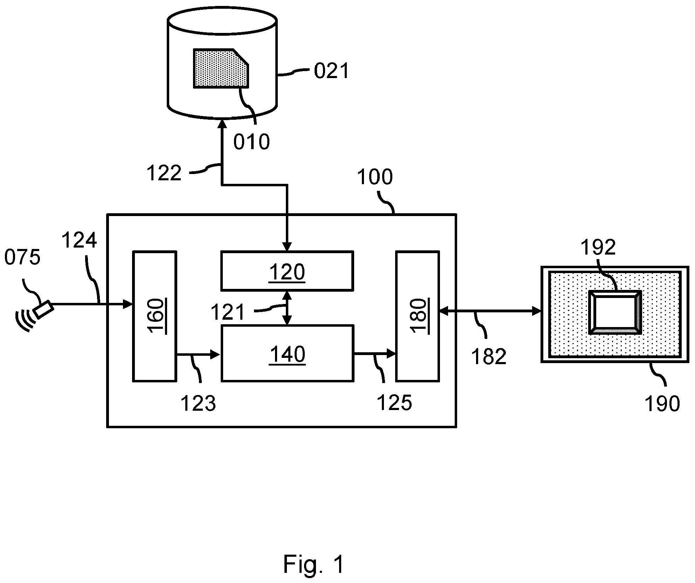

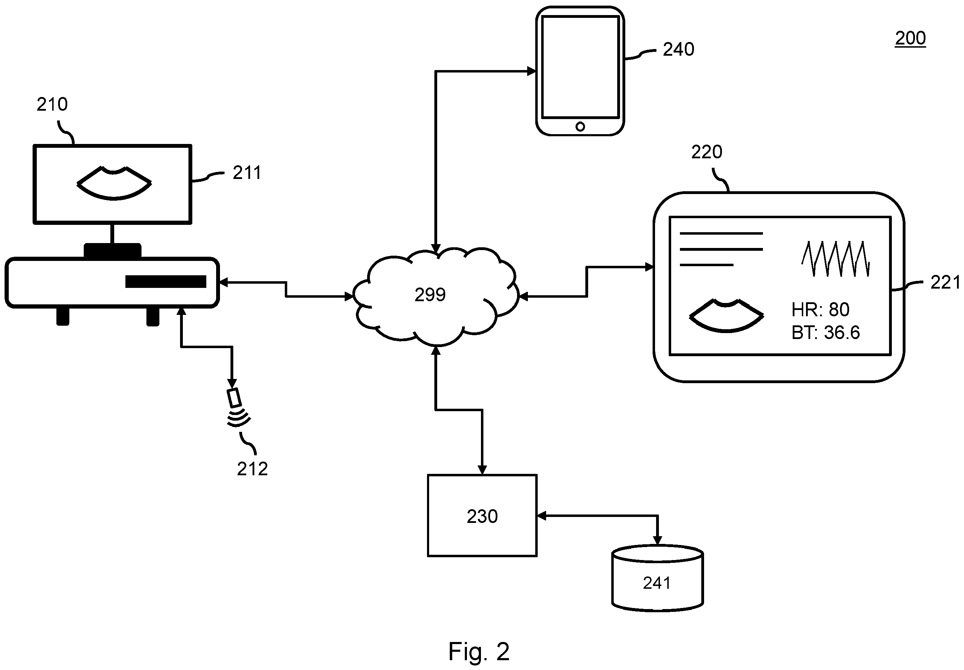

**Fig. 1** shows a customization unit 100. The customization unit 100 may be for use in an ultrasound system as described herein, e.g., ultrasound system 200 of Fig. 2. The ultrasound system may be operable by one or more users of the ultrasound system to process ultrasound measurements of a patient, and the system may comprise an ultrasound unit which is operable by a user to perform the ultrasound measurements of the patient. As also discussed with respect to Fig. 2, the customization unit 100 may be separate unit, e.g., a separate device, of the ultrasound system; but the customization unit 100 can be combined with an existing unit of the ultrasound system. For example, customization unit 100 can be the ultrasound unit, a patient monitoring unit, or a bridge unit of the ultrasound system. The customization unit may also be referred to as a customization system or, in the context of the ultrasound system, as a customization subsystem.

The customization unit 100 may comprise a data interface 120 for accessing interaction data 010 of one or more interactions of a current user performing a current task, e.g., at the ultrasound unit or other unit in which the customization unit 100 is integrated, or at a different unit of the ultrasound system. User-facing functionality for performing the current task may be provided at multiple different skill levels. The data interface 120 may instead or in addition be used to access various other data, in particular model data of a machine learning model trained to determine a real-time skill estimate also described elsewhere.

For example, as also illustrated in Fig. 1, the input interface may be constituted by a data storage interface 120 which may access the data 010 from a data storage 021. For example, the data storage interface 120 may be a memory interface or a persistent storage interface, e.g., a hard disk or an SSD interface, but also a personal, local or wide area network interface such as a Bluetooth, Zigbee or Wi-Fi interface or an ethernet or fibreoptic interface. The data storage 021 may be an internal data storage of the system 100, such as a hard drive or SSD, but also an external data storage, e.g., a network-accessible data storage. For example, at least part of the interaction data 010 may be accessed from log files of the ultrasound unit of the ultrasound system, for example via a network-accessible data storage on which the log files are stored. In some embodiments, respective data, e.g., respective parts of the interaction data, may each be accessed from a different data storage, e.g., via a different subsystem of data storage interface 120. Each subsystem may be of a type as is described above for the data storage interface 120.

The customization unit 100 may further comprise a processor subsystem 140 which may be configured to, during operation of the system 100, from the user interaction data 010, determine a real-time skill estimate of the current user. The real-time skill estimate may indicate a current ability of the current user to perform the current task.

The processor subsystem 140 may be further configured to customize the ultrasound system for performing the current task at a skill level corresponding to the real-time skill estimate of the current user. In particular, the customization unit 100 may comprise an output interface (not shown) for outputting, to a user interface component for the current task, an instruction to provide the user interface at this skill level. The output interface may provide the instruction via internal data communication or via external data communication. For example, the output interface may be implemented as a communication interface as further discussed below. As another possibility, the output interface may be constituted by the data interface 120.

As illustrated in the figure, the customization unit 100 may optionally comprise a sensor interface 160 for accessing sensor data 124 acquired by one or more sensors 075. In particular, the customization unit may be incorporated in the ultrasound unit 100, in which case the sensor interface 160 may at least be for accessing ultrasound measurements 124. The sensor interface may instead or in addition be for accessing sensor measurements apart from the ultrasound measurements, for example, from one or more sensors located on an ultrasound prove and providing sensor data indicating how the probe is used, for example, an accelerometer, a gyroscope, a pressure sensor, an optical sensor, and/or a temperature sensor. As another example, the customization unit may be incorporated in a patient monitoring unit, in which case the sensor interface 160 may be for accessing sensor data from sensors measuring one or more vital signs, for example. The sensor(s) 072 can but do not need to be part of the unit 100. The sensor interface 160 may have any suitable form corresponding in type to the type of sensor, including but not limited to a low-level communication interface, e.g., based on I2C or SPI data communication, or a data storage interface of a type as described above for the data interface 120.

The customization unit 100 may also comprise a communication interface (not shown) configured for communication with other unit(s) of the ultrasound system. The communication interface may be arranged for direct communication with the other unit(s), e.g., using USB, IEEE 1394, or similar interfaces. The communication interface may also communicate over a computer network, for example, a wireless personal area network, an internet, an intranet, a LAN, a WLAN, etc. For instance, the communication interface may comprise a connector, e.g., a wireless connector, an Ethernet connector, a Wi-Fi, 4G or 4G antenna, a ZigBee chip, etc., as appropriate for the computer network. The communication interface may also be an internal communication interface, e.g., a bus, an API, a storage interface, etc.

The customization unit 100 may further comprise an I/O interface 180 for communication 182 with one or more input/and or output devices for enabling communication with a user. Examples of input devices may include, but are not limited to, a microphone, a keyboard, a pointing device such as a mouse or a touch screen, a game controller, a Bluetooth controller, a VR controller, and a gesture-based input device, or the like. Examples of output devices may include, but are not limited to, a monitor or display, speakers, or the like. The processor subsystem 140 may configured to provide user-facing functionality, e.g., a user interface, to a user, via the I/O interface 180. This is illustrated in the figure by showing a display 190 on which a user interface element 192 is shown that may be customized according to a determined real-time skill estimate. Generally, the input and/or output devices may be incorporated in, or connectable to, the customization unit 100.

In general, each system described in this specification, including but not limited to the system 100 of Fig. 1, may be embodied as, or in, a single device or apparatus, such as a workstation or a server. The device may be an embedded device. The device or apparatus may comprise one or more microprocessors which execute appropriate software. For example, the processor subsystem of the respective system may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the processor subsystem of the respective system may be implemented in the device or apparatus in the form of programmable logic, e.g., as a Field-Programmable Gate Array (FPGA). In general, each functional unit of the respective system may be implemented in the form of a circuit. The respective system may also be implemented in a distributed manner, e.g., involving different devices or apparatuses, such as distributed local or cloud-based servers.

Fig. 2 shows an ultrasound system 200. The ultrasound system 200 may be operable by one or more users of the ultrasound system 200 to process ultrasound measurements of a patient. The ultrasound system 200 may generally comprise various units that may be used at various stages of the processing of the ultrasound images, e.g., in preparation, image acquisition, and/or analysis. The figure shows several such units that may be used separately or in combination. Generally, each of the described unit may be implemented as described with respect to Fig. 1.

In particular, the ultrasound system 200 may comprise an ultrasound unit 210. Such an ultrasound unit may also be known as an ultrasound device or as a medical ultrasound device. The ultrasound unit 210 may be operable, by a user of the ultrasound system, to perform the ultrasound measurements of the patient. The ultrasound unit 210 may be used for the preparation and/or image acquisition stages of ultrasound use. Various types of ultrasound units are in use today and may be adapted as described herein. Shown in the figure is a portable ultrasound device, in this case with wheels, as is on the market today. Examples of such devices include the Philips EPIQ line of devices, e.g., EPIQ Elite and EPIQ CVx^{∗}, or the Philips Affiniti series. Another series of device that may be used are wired or wireless carriable ultrasound devices, such as the Philips CX50 series or Philips Lumify. Generally, an ultrasound unit 210 may comprise a probe 212 for obtaining ultrasound measurements, for example, using one or more CMUT and/or piezoelectric transducers, and input and/or output devices for providing a user interface to a user for preparing the scan and inspecting the ultrasound measurements, for example a display 211. When in image acquisition mode, the ultrasound unit 210 may be configured to continually capture ultrasound images and/or derive measurements from them, for example to derive new measurements at a period of at most or at least 1 second, at most or at least 10 seconds, or at most or at last 1 minute, or at most or at least 10 minutes, and to show them on the screen 211 and/or store them.

As shown in the figure, the ultrasound system 200 may further comprise a patient monitoring unit 220, in other words, a patient monitor. The patient monitor 220 may be configured to show, on a display 221, information based on the performed ultrasound measurements, for example, the ultrasound images captured by the ultrasound unit 210, or measurements derived from it, such as a measured anatomic dimension, e.g., a measured volume of a body part, etc. The patient monitor 220 may be used for the analysis stage of ultrasound use. As also illustrated in the figure, the patient monitor is generally configured to show patient information and/or measurements apart from those provided by the ultrasound unit 210 as well, e.g., measurements of vital signs such as a heart rate and/or body temperature. A system 200 that comprises both an ultrasound unit 210 and a patient monitor 220 on which data from the ultrasound unit 210 is shown, may generally be referred to as an ultrasound for monitoring, US4UM, system.

The system 200 may be for use for example in a critical care and/or perioperative environment, e.g., in an intensive care unit. However, the system 200 may also be used in other clinical set-ups, which may also involve multiple users who have potentially different goals to accomplish with ultrasound. Another envisaged use is use of an US4M system in a sonography clinic. In this example, sonographers may for example be assigned to specific tasks and/or exams based on their experience level. Also in such cases, detecting the experience level and/or task automatically and customizing the ultrasound system accordingly, becomes relevant and useful.

Another unit that can optionally be part of the ultrasound system 200, is a bridge device 230. The bridge device 230 may facilitate information exchange between the ultrasound unit 210 and the patient monitor 220. For example, the bridge device may provide a shared storage 241, and may optionally provide functionality such as image processing, data retrieval, data storage, and/or sensor control. The ultrasound unit 210 may optionally be configured to store, on the storage 241, data such as ultrasound measurements and/or log files, and from which the patient monitor 220 may retrieve the data. The bridge device 230 may optionally be configured to store, on the storage 241, information collected from the patient monitor 220 and passed to the ultrasound unit 210, such as but not limited to, patient demographics and vital signs. The bridge device 230 may optionally provide a user interface for facilitating information exchange, e.g., for accessing prior stored data and/or reviewing previously stored instructions or imaging orders. The bridge device 230 may be used for the image acquisition and/or analysis stage of ultrasound use. For example, during image acquisition, the bridge device may be used for scan guidance, e.g., to invoke prior exams and/or views from storage 241. During analysis, the bridge device 230 may for example be used to push images and/or loops to the bridge device 230 and analyse them on this device.

Another example of a unit that can optionally be part of the ultrasound system 200, is an inspection device 240 that may allow inspection of the ultrasound measurements of the ultrasound unit 210 by a user. As illustrated in the figure, the inspection device 240 may be a mobile device, e.g., a smartphone, a tablet, smart glasses, a virtual reality headset, a hologram, or the like. Such an inspection device may be useful in particular for use at a point of care outside of a hospital, for example, at home, in an ambulance, or at a sports facility. These are typical examples where the amount of experience may differ greatly between users, so that customization is beneficial.

Also shown in the figure is a computer network 299 over which the different devices of the ultrasound system 200 may be configured to interact. The computer network can be the internet or an internal communication network of a hospital, for example.

The ultrasound system 200 may provide user-facing functionality for performing a current task at one of the units of the ultrasound system, e.g., at the ultrasound unit 210; at the patient monitor 220; at the bridge device 230; or at the inspection device 240. A task may be related to performing a particular measurement or imaging a particular body part and/or at a particular view, for example. Various examples of such tasks are provided throughout this specification. The user-facing functionality may be provided at multiple different skill levels, for example, at multiple different skill levels for each of a set of multiple different roles. For example, the system may provide the functionality at at least two or at least three different skill levels, or at least two or three levels for a given role.

Interestingly, the ultrasound system 200 may comprise a customization unit. The customization unit may be as discussed with respect to Fig. 1. The customization unit may serve to let the ultrasound system 200 provide the user-facing functionality for the current ask at the right skill level for the situation at hand. To this end, the customization unit may be configured to obtain user interaction data of one or more interactions of a current user performing the current task at the ultrasound system 200. The customization unit may be further configured to, from the user interaction data, determine a real-time skill estimate of the current user. The real-time skill estimate may indicate a current ability of the current user to perform the current task. The customization unit may be further configured to derive from the user interaction data the current task that the current user is performing, but may also derive the current task by other means. The customization unit may then be configured to customize the ultrasound system for performing the current task at a skill level corresponding to the real-time skill estimate of the current user.

Generally, the customization unit may be integrated in one of the previously discussed units, e.g., may be combined with the ultrasound unit 210; the patient monitor 220; the bridge device 230; or the inspection device 240. However, the customization unit may also be implemented as a separate device. The customization unit may comprise a user interface configured to enable the user to learn and/or modify the skill level at which the ultrasound system is customized, in other words, to provide a manual override. This user interface is preferably provided at the device that also provides the user-facing functionality.

Depending on how the customization unit is implemented, the customization unit may obtain the user interaction data by internal or external data communication. For example, the user interaction data may comprise or be based on device logs, e.g., of the ultrasound unit 210, e.g., stored at a shared storage 241. Another possibility is that the customization unit is implemented at the same device that provides the user-facing functionality, in which case the user interaction data may be obtained by internally accessing a log file, by receiving a callback from the user-facing functionality, by using an accessibility API to introspect the user interface, or the like.

Depending on how the customization unit is implemented, the customization unit may also customize the ultrasound system in various ways, for example, using internal or external data communication. For example, the ultrasound unit 210 or another unit may be configurable to operate at a given skill level via an externally accessible API such as a REST API, or may periodically check a setting of the given skill level at a shared storage 241, etc.

The customization unit may be configured to determine the real-time skill estimate independent from any user identification. Although a user identification may be performed in ultrasound system 200, for example for authorization purposes and/or to derive the role of the current user, interestingly, the real-time skill estimate may be determined independently from it. As also discussed elsewhere, this can have various advantages including improved privacy and better adaptation to the current context.

One or more of the units of system 200, for example, the ultrasound unit and/or the patient monitor 220, may comprise an expert guidance subsystem, for example a system for providing remote expert guidance based on video and/or audio, also known as a tele-health-based support interface. Data from such a subsystem may be used for example to derive the current task, and interactions with the subsystem may be captured as user interaction data, e.g., use of expert guidance may imply a lower real-time skill estimate.

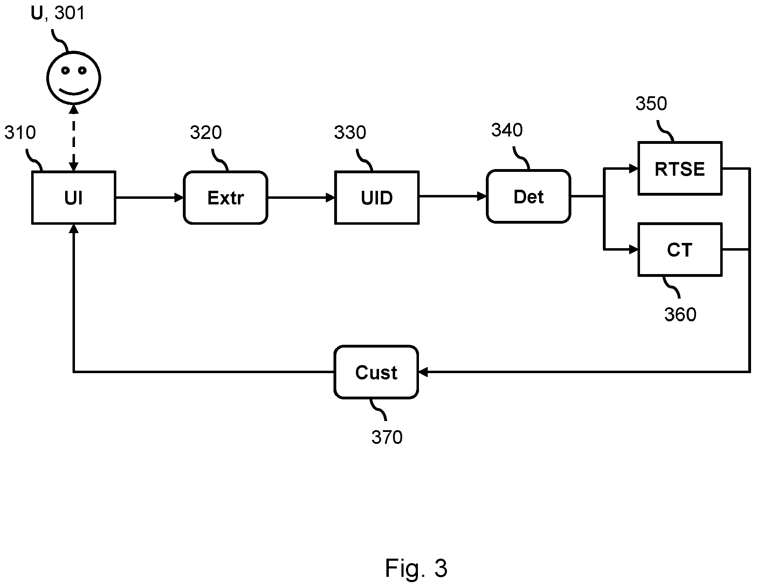

**Fig. 3** shows a detailed, yet non-limiting, example of how to customize an ultrasound system. The ultrasound system may be operable by one or more users of the ultrasound system to process ultrasound measurements of a patient. For example, the techniques described with respect to this figure may be implemented by a customization unit as described with respect to Fig. 1 and/or Fig. 2.

Generally, the customization of the ultrasound system may be performed by leveraging user interactions with units of the ultrasound system to estimate a profile of the user; in particular, their real-time skill estimate which may indicate an experience level and/or a current physical and/or mental capability to perform the task at hand, with the goal to use this information to provide customize the ultrasound system for the current user, for example, by providing relevant assistance to the user and/or automatically carry out workflow-simplifying actions on one or more of the system components.

Such a customization may involve one or more of the following steps, which are discussed in more detail below:
1) Identifying of the current stage of the processing of the ultrasound measurements, e.g., ultrasound scan preparation, image acquisition, analysis of the ultrasound measurements, etc.;
2) Using user interactions with the system, preferably from the current stage of the workflow, to estimate the profile of the user, in particular their real-time skill;
3) Optionally, refining the user profile estimate by incorporating historical information for that user;
4) Estimating of the current task on hand; and
5) Given the user profile and task-to-be-performed, customizing the ultrasound system by e.g. providing relevant assistance to the user and/or automate one or more aspects of the workflow.

Below, examples are given of how the above steps may be implemented:

### 1) Identification of the stage of the workflow

Generally, a processing workflow for ultrasound measurements may be divided into respective stages. The tasks of a given stage may typically be performed by a single user and/or at a single unit of the ultrasound device. Accordingly, a real-time skill estimate may be determined as described herein for a current stage, from interactions of the current user only at this current stage, possibly using a real-time skill estimate and/or role derived at an earlier stage as an additional input.

Typically, a clinical workflow involving an ultrasound scan may start with the user prepping the US system, e.g., by changing modes, establishing pre-sets etc. Once the desired settings have been set, the workflow may proceed to the image acquisition stage. Once the desired images have been acquired, various types of analysis (e.g., quantification or other analytic processing) may be performed on scanned images.

Generally, the stage of the workflow may be identified based on the use of corresponding functionality at the ultrasound system, as indicated for example by interaction data at respective units of the ultrasound unit. For example, a transition from the image acquisition to the analysis stage may be initiated by transferring ultrasound images and/or measurements from an ultrasound unit to a patient monitoring unit via a study/image export or other button functionality on the ultrasound unit. Based on these button presses and other system transitions in the system, a current stage of the clinical workflow may be ascertained.

### 2) Evaluation of user interaction with system

Generally, the use of an ultrasound system, such as a US4M (ultrasound for monitoring) system, may include user interactions during the various stages, many of which may be leveraged to estimate the profile and/or capabilities of the user. This is illustrated in the figure, which schematically shows a user interface **UI,** 310, that is used by the ultrasound system to provide user-facing functionality to a user **U,** 301, for performing a current task at the ultrasound system. The figure further shows an extraction operation **Extr,** 320, in which user interaction data **UID,** 330, may be obtained of one or more interactions of the user U with the ultrasound system. Examples of user interactions on the ultrasound unit that may be represented by the user interaction data **UID** may include badge/fingerprint scans, button presses, scanning mode selection, accessing prior images etc. Interactions on the bridge/patient monitor can include reviewing imaging orders set by experts and accessing prior data on that patient, etc.

The figure also shows a determination operation **Det,** 340, in which, from the user interaction data **UID,** 330, a real-time skill estimate **RTSE,** 350 of the current user **U** may be determined. The real-time skill estimate **RTSE** may indicate a current ability of the current user to perform the current task. Once the workflow stage has been identified, for that step in the workflow, it may be evaluated whether one or more user interactions with the system can be utilized to estimate the profile of the user. Preferably, only interaction data from the current stage is used to determine the real-time skill estimate, so that a customization can be provided directed towards the current user in the current stage. The interaction data may comprise items representative of individual interactions and/or aggregate features over multiple interactions.

For example, if the workflow is currently in the scan preparation stage, examples of user interaction may include:
- sequence and/or timing of button presses on the ultrasound unit, for example, represented as button press counts per button, or encoded by a variational autoencoder;
- scanning mode used to begin the scan, for example, represented as a categorical variable;

For example, if the workflow is in the image acquisition stage, examples of user interactions may include:
- image scanning patterns, including what intermediate images/features are passed through before settling on the image-of-interest, for example, represented by a variational autoencoder, cluster identities for the intermediate images according to a clustering algorithm, or manually configured features such as a count of intermediate images per type or a time to achieve a target view;
- data representing usage patterns of the ultrasound probe, collected from available sensors on the ultrasound unit itself or elsewhere in the ultrasound system, for example, movement patterns (represented as a signal or as signal features, e.g., a 2D signal such as an image; image features; a ID signal, or otherwise); inserted pressure; or body area of scanning;
- one or more metrics derived from individual interactions, like time-to-obtain first image, or how many images the user saves;
- accessing of prior scans to use as a reference for obtaining the right image, represented for example as a number of accesses;
- application of image quality (IQ) estimation methods to estimate quality of the initially acquired images, represented for example as a bit map of which methods have been applied;

For example, if the workflow is in the analysis stage (including image interpretation, data curation, and/or data transfer), examples of user interaction include:
- quantifications/measurement performed on the saved images, for example represented as a number and/or type of actions performed;
- if auto-quantification approaches are used, manual edits performed on the results of the auto-quantification, for example represented as a number and/or kind of manual edits, e.g., zooming and/or unzooming actions;
- accessing of prior scans to use as a reference for obtaining the right quantifications, represented e.g. as a number of accesses;
- analyses run on the previously acquired images, and features extracted from these analyses, e.g., initiated by the user (features may include a type of analysis and a result of the previous analysis), or automated (features may include a result of the previous analysis);
- images curated/chosen by the user to send to a patient monitor, e.g. represented as a number of images specified by an earlier expert user and/or a number of images not so specified;
- user interactions on the transferred images on the patient monitor, represented, for example, as a number and/or type of operations performed.

The above user interaction data may be augmented by context information about the patient being imaged and/or the physical environment in which the ultrasound system is being used. Also product information of the ultrasound system may be used, e.g., hardware and/or software information such as device type, manufacturer, version, year of make, etc.

For example, the context information may comprise features representing a role and/or number of people present at the ultrasound system, a workflow load and/or schedule and/or activities prior to ultrasound usage of the user, a location of the ultrasound system, an air quality, a noise level, a time of day, or a time with respect to start of shift. Such aspects may influence physical and/or psychological state of the user, and may thus influence real-time skill levels.

For example, the information about the patient being imaged may be indicative of a difficulty of imaging the patient. Patients that are inherent difficult to image, or whose current condition precludes easy image acquisition, may be considered as such, and this may be used to appropriately weight the previously mentioned user interaction data. More generally, using patient information as an additional input may be used to better recognize patterns that a person of a particular level of training follows compared to someone of a different level or category of training.

The above data may be leveraged to determine, **Det** the estimate of the user/user category/user experience level **RTSE.** This estimate, or the skill level derived from it, may optionally be displayed in a user interface. In an embodiment, the real-time skill level may be determined from interaction data that comprises at least 5, at least 10, or at least 50 interaction items. The interaction data may be augmented by a feature vector of at least 2, at least 5, or at least 10 features of context information, for example. Generally, the determined real-time skill estimate **RTSE** may be expressed as a discrete category, e.g., notice, expert; or on a continuous scale (e.g., from 0 to 100).

In an embodiment, the real-time skill estimate **RTSE** may be determined using a trained machine learning model. An advantage of using machine learning is that generally, a high accuracy may be achieved. Another advantage is that the learning can be automated and continuously updated, and that an explicit definition of features to be extracted from interaction data may be avoided. The model may receive as input the interaction data and/or context information. Individual interaction items may be represented as respective feature vectors. For example, the feature vector may comprise features corresponding to the respective types of interaction items discussed above, where a feature vector of a particular type of interaction item has nonzero feature vectors only for the features corresponding to its type. The respective feature vectors can be input into the machine learning model in aggregated form, e.g., by summing the respective feature vectors, or sequentially, e.g., by using a recurrent model such as a recurrent neural network.

The machine learning model may be parameterized by a set of parameters. For example, the model may be a neural network, a support vector machine, a random forest, etc. Neural networks are also known as artificial neural networks. Examples include deep neural networks and convolutional neural networks. In this case, the set of parameters may comprise weights of nodes of the neural network. For example, the number of layers of the model may be at least 5 or at least 10, and the number of nodes and/or weights may be at least 1000 or at least 10000. Various known architectures for neural networks and other types of machine learnable models may be used.

Training of the machine learning model may be performed using stochastic approaches such as stochastic gradient descent, e.g., using the Adam optimizer as disclosed in Kingma and Ba, "Adam: A Method for Stochastic Optimization" (available at https://arxiv.org/abs/1412.6980 and incorporated herein by reference). As is known, such optimization methods may be heuristic and/or arrive at a local optimum. Training may be performed on an instance-by-instance basis or in batches, e.g., of at most or at least 64 or at most or at least 256 instances. The training of the model may comprise applying the model to obtain a model output, and optimizing the trainable parameters of the model based on the model output, for example, to optimize a loss function based on the determined and the actual model output. The training may be performed based on labelled training data by a separate training system, based for example on the hardware configuration of Fig. 1.

As an alternative to using a trained model for the determination **Det,** it is also possible to use a rule-based system e.g. with manually defined rules. For example, respective real-time skill scores may be assigned to respective interaction items according to a respective mapping for the respective type of interaction item. This mapping can be manually defined. The real-time skill score can for example be a binary score (indicating or not indicating high real-time skill), or more generally a score on a N-point scale (indicating respective degrees of real-time skill), where N=3 or N=5 for example. As an illustrative example, for time-to-obtain first image T, a score indicating high real-time skill may be assigned if T<10 seconds; a score indicating low real-time skill may be assigned if T>1 minutes; and otherwise a score indicating medium real-time skill may be assigned. The thresholds may be manually defined but can also be automatically determined based on training data. Respective real-time skill scores may be aggregated to determine the overall real-time skill estimate, e.g., as an average or the like. An advantage of using a rule-based system is that interpretability is improved, e.g., it is easier to visualize/understand for a human how a certain conclusion was reached.

In some embodiments, the determination of the real-time still estimate **RTSE** also involves determining a degree of confidence in the output. For example, a machine learning model may output the degree of confidence as known per se, e.g., in the form of a classification score. For a rule-based system, the degree of confidence may be based on a variance of determine real-time skill scores, for example. For example, the degree of confidence may be used by only customizing the ultrasound system according to the real-time skill estimate **RTSE** if the confidence exceeds a given threshold.

In some embodiments, the determined real-time skill estimate **RTSE** may be specific for a particular role that the user has (e.g., intensivist, clinician, resident, nurse, etc.), e.g., with respect to the treatment of the current patient. The user-facing functionality may be for performing a current task at multiple different skill levels and according to multiple different roles. In such cases, for example, a respective trained model or a respective rule-based system may be used to determine a real-time skill estimate specific to that respective role, and the role-specific real-time skill estimate may then be used to customize the ultrasound system, as also described elsewhere.

### 3) Incorporating historical trends to increase confidence in user profile estimate

In typical embodiments, the real-time skill estimate **RTSE** is determined independently from any user identification. In some embodiments, for example embodiments where a user identification is already in place anyway, it is however possible to employ such user identification to refine the real-time skill estimate.

In particular, the user may be identified and the determined real-time skill estimate **RTSE** may be corroborated according to user profile data of the user. For example, once the initial estimation **RTSE** of the user has been accomplished, the confidence in that estimation may be increased by polling a source of patient data, e.g., an institutional databases or a US4M database as applicable, for historical information related to that user. For example, if the initial estimate indicates a certain level of experience/expertise (e.g., based on a sequence of button pushes), the historical data for that level of experience and/or expertise may be polled to look for a close match with a specific user. A match may increase confidence in the determined real-time skill estimate **RTSE,** e.g. the degree of confidence as discussed above may be increased.

In case where user identification is performed, the user profile may also be updated based on the determined real-time skill estimate, e.g., a skill level stored in the user profile may be updated. Generally, as users progress in experience level over time, the provided techniques cause the ultrasound system to change the type of feedback provided to that user due to the changing real-time skill estimate **RTSE.** This information can then also be used to update the user profile. Accordingly, the user expertise level in the profile may be gradually estimated. For instance, at the beginning the system starts with full automation mode, and as data is being gathered the level of automation and feedback may change based on the real-time skill estimate.

Identification may be performed using conventional means, e.g., by obtaining a user identifier from a scanned badge. Alternatively, it may also be possible to identify the user based on user interaction data **UID,** e.g., based on usage patterns such as a sequence of button presses (e.g., users by force of habit often use a specific combination of settings to scan) and/or a selected scan mode (e.g., users often follow same sequence of actions to get to desired system state), and/or based on sensor measurements, e.g., of a camera and/or microphone. This way, there is no need for a manual identification step by the user. When there is no manual identification by the user, and an identification has been made, the user may be asked, via a user interface, to confirm the identification. Optionally, the user profile associated with an identified user may indicate whether the user has opted in or out of being profiled for the purposes of customizing the ultrasound system. This way, only information about users who have opted in, may be presented via the user interface, improving privacy.

### 4) Estimating current task

The customization unit may obtain the current task **CT,** 360 that is being performed and whose execution may be customized, in various ways. In some embodiments, for example, the current task may be manually selected and/or explicitly initiated by the user via the user interface.

In other embodiments, as also illustrated in the figure, the customization unit may be configured to derive from the user interaction data **UID** the current task **CT** that the current user is performing. The current task may be estimated for example based on analysing initial images from the scan and/or from a sequence of button presses and system transitions of the user performing the task.

To determine the current task **CT,** the customization unit may further use data captured from an expert guidance subsystem of the ultrasound system, such as the Reacts framework or similar. For example, an expert user may be located in a remote location and may guide a user located at the bedside who has been determined to have a low real-time skill estimate. The system may in such a case complement the guidance provided by the expert user, for example by presenting the guidance in a way that is easy to interpret for the bedside user, or by augmenting the guidance with data or information that is relevant to the task on hand. For example, if the remote expert user suggests the acquisition of an A4C image, then the system may pull up the acquisition guidance module for A4C acquisition and/or prior A4C images that can provide a template for the inexperienced bedside user.

### 5) Providing relevant assistance and/or workflow simplification

Based on the real-time skill estimate **RTSE** and the current task **CT,** a customization operation **Cust,** 370, may customize the ultrasound system for performing the current task at a skill level corresponding to the real-time skill estimate of the current user. The customization may comprise, depending on the skill level, providing or not providing a user guidance. The customization may further comprise, depending on the skill level, automating or not automating an operation. The customization may optionally further take into account the role (e.g.: lead intensivist, junior clinician, resident, nurse, GP, midwife, sports medic) of the user to perform the customization and provide the user-facing functionality accordingly. Similarly to the current task **CT,** the current role may be derived in various ways, e.g., from the user interaction data **UID;** or by manual selection.

Some specific examples of customizations are now provided.

In one example, depending on the skill level, the customization unit may automatically retrieve and show, or not automatically retrieve and show, information about a prior scan for a current follow-up scan. For example, the system may determine that the current task is associated with a clinical order for the follow-up scan and identify the prior scan from the clinical order. The information may be tagged with user information.

In one example, depending on the skill level, the customization unit may automatically pre-load, or not automatically pre-load, scan settings suitable for the current task. For example, the current task may relate to line placement, in which case line placement settings may be pre-loaded, for example, system settings for device visualization, such as tissue-specific presets. For example, the settings may include one or more of depth, focus, harmonics, NeedleViz mode activation, transmitting frequency, gain, and dynamic range. Line placement may be detected automatically e.g., by identifying a needle, wire, or sheath in the ultrasound images. This customization may be performed in particular if the current user is a nurse.

In one example, the current task may relate to obtaining a given view of an organ. The customization unit may in such a case, depending on the skill level, show or not show example images of the given view of the organ, e.g., schematic images or images of actual previous scans. For example, if the initial images from a scan indicate that the user is trying to obtain apical views of the heart (e.g., A2C/A4C), then the ultrasound system may be configured to provide assistance by pulling up examples of A2C/A4C views from prior patients that match the body habitus of the current patient.

In one example, depending on the skill level, the customization unit may automatically retrieve one or more relevant ultrasound images and/or measurements from a database.

In one example, depending on the skill level, the customization unit may or may not provide feedback on a status of an ultrasound scan being performed.

In one example, depending on the skill level, the customization unit may or may not activate guidance in the form of assistance provided by a tele-health application, and/or to determine an assistance characteristics (e.g., type and/or intensity) of the tele-health application.

In embodiments where user identification is performed, the customization unit may be configured to customize the ultrasound system according the user profile. In particular, customization **Cust** may use manually provided customization settings. For example, nurse#1 can program the system to transition itself to certain settings that they prefer, once it identifies that the current user is nurse#1. In another example, the head intensivist can decide what feedback needs to be provided by any user classified as a "junior resident". Customization **Cust** may also use usage patterns learned about the user. This way, the system may learn to provide user-specific assistance based on knowing what that user has needed in the past when doing similar scans. For example, if a user consistently ignores or closes a certain guidance, the system may be configured not to provide that guidance the next time the user performs the same task.

Generally, the extraction **Extr,** the determination **Det** and the customization **Cust** may be performed continuously in the background, e.g., the determination **Det** and customization **Cust** may be performed periodically and/or upon availability of new user interaction data **UID.** In particular, for a given user, a first real-time skill estimate **RTSE** may be updated to an updated real-time skill estimate based on new user interaction data, for example, may be updated once or more than once. Customization **Cust** may for example be performed once the real-time skill estimate **RTSE** is determined with a confidence exceeding a threshold. Customization may be performed only once for a given task, but it is also possible to perform the customization multiple times as updated estimates of the real-time skill estimate **RTSE** are determined. When the user has manually modified the skill level, typically no further automatic customization for the current task is performed.

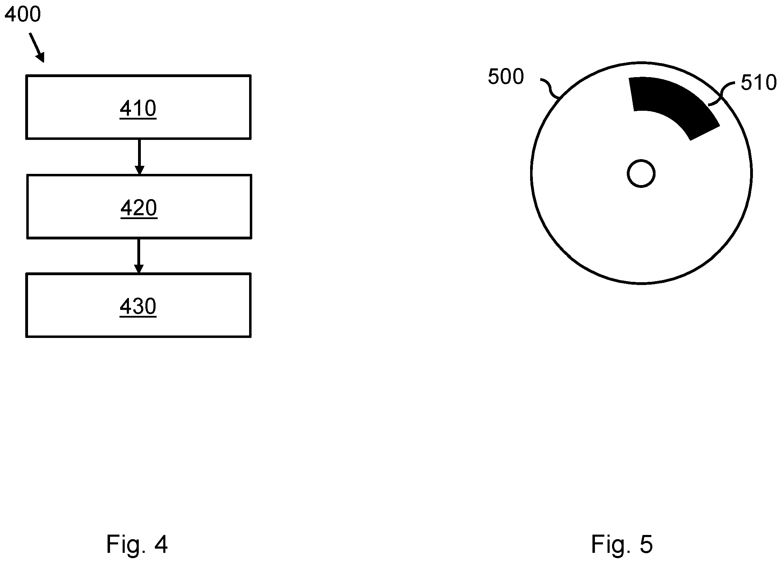

**Fig. 4** shows a block-diagram of computer-implemented method 400 of customizing an ultrasound system. The ultrasound system may be operable by one or more users of the ultrasound system to process ultrasound measurements of a patient. The ultrasound system may comprise an ultrasound unit operable to perform the ultrasound measurements of the patient. The method 400 may correspond to an operation of the customization unit 100 of Fig. 1 and/or the ultrasound system 200 of Fig. 2. However, this is not a limitation, in that the method 400 may also be performed using another system, apparatus or device.

The method 400 may comprise, in an operation titled "OBTAIN USER INTERACTIONS", obtaining 410 user interaction data of one or more interactions of a current user performing a current task at the ultrasound system. The ultrasound system may provide user-facing functionality for performing the current task at multiple different skill levels.

The method 400 may comprise, in an operation titled "ESTIMATE REAL-TIME SKILL", from the user interaction data, determining 420 a real-time skill estimate of the current user. The real-time skill estimate may indicate a current ability of the current user to perform the current task.

The method 400 may comprise, in an operation titled "CUSTOMIZE", customizing 430 the ultrasound system for performing the current task at a skill level corresponding to the real-time skill estimate of the current user.

It will be appreciated that, in general, the operations of method 400 of Fig. 4 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 5****,** instructions for the computer, e.g., executable code, may be stored on a computer readable medium 500, e.g., in the form of a series 510 of machine-readable physical marks and/or as a series of elements having different electrical, e.g., magnetic, or optical properties or values. The medium 500 may be transitory or non-transitory. Examples of computer readable mediums include memory devices, optical storage devices, integrated circuits, servers, online software, etc. Fig. 5 shows an optical disc 500. Alternatively, the computer readable medium 500 may comprise data 510 representing a machine learnable model trained to determine a real-time skill estimate from user interaction data.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. An ultrasound system (200), wherein the ultrasound system is operable by one or more users of the ultrasound system to process ultrasound measurements of a patient, the system comprising:
- an ultrasound unit (210), wherein the ultrasound unit is operable to perform the ultrasound measurements of the patient;
- a customization unit (210, 220, 230, 240) configured to:
- obtain user interaction data of one or more interactions of a current user performing a current task at the ultrasound system, wherein the ultrasound system provides user-facing functionality for performing the current task at multiple different skill levels;
- from the user interaction data, determine a real-time skill estimate of the current user, wherein the real-time skill estimate indicates a current ability of the current user to perform the current task;
- customize the ultrasound system for performing the current task at a skill level corresponding to the real-time skill estimate of the current user.

2. The ultrasound system (200) of claim 1, wherein the customization unit is configured to determine the real-time skill estimate independent from any user identification.

3. The ultrasound system (200) of any preceding claim, wherein the ultrasound system is an ultrasound for monitoring system, wherein the ultrasound for monitoring system further comprises a patient monitoring unit configured to show information based on the performed ultrasound measurements,
and wherein the user performs the current task at the patient monitoring unit or at a bridge device of the ultrasound for monitoring system used for information exchange between the ultrasound unit and the patient monitoring unit.

4. The ultrasound system (200) of any preceding claim, wherein the user-facing functionality is for performing the current task at multiple different skill levels and according to multiple different roles.

5. The ultrasound system (200) of any preceding claim, wherein the customization unit is further configured to derive from the user interaction data the current task that the current user is performing.

6. The ultrasound system (200) of any preceding claim, wherein the current task is part of a current stage of multiple stages of the processing of the ultrasound measurements, wherein the real-time skill estimate is determined for the current stage from interactions of the current user only at the current stage, optionally using a real-time skill estimate and/or role derived at an earlier stage.

7. The ultrasound system (200) of any preceding claim, wherein the user interaction data comprises multiple interaction items, comprising one or more interaction items representing individual interactions of the user in performing the current task and/or one or more interaction items representing aggregate quantities determined over multiple interactions of the user with the system in performing the current task and/or one or more interaction items representing sensor measurements of the ultrasound system apart from the ultrasound measurements by the ultrasound unit.

8. The ultrasound system (200) of any preceding claim, wherein the customization unit is configured to determine the real-time skill estimate based on context information about the patient being imaged and/or the physical environment in which the ultrasound system is being used.

9. The ultrasound system (200) of any preceding claim, wherein the customization unit is configured to customize the ultrasound system by, depending on the skill level, providing or not providing a user guidance, and/or, depending on the skill level, automating or not automating an operation.

10. The ultrasound system (200) of any preceding claim, wherein the customization unit comprises a user interface configured to enable the user to learn and/or modify the skill level at which the ultrasound system is customized.

11. The ultrasound system (200) of any one of the preceding claims, wherein the ultrasound system is for use in a critical care and/or perioperative environment.

12. The ultrasound system (200) of any one of claims 1-10, wherein the ultrasound system is for use outside of a hospital, for example, at home, in an ambulance, or at a sports facility.

13. A customization unit (100) for use in the ultrasound system according to any one of the preceding claims, wherein the customization unit comprises the processor subsystem (140) configured to obtain the user interaction data (010); determine the real-time skill estimate; and customize the ultrasound system.

14. A computer-implemented method (400) of customizing an ultrasound system, wherein the ultrasound system is operable by one or more users of the ultrasound system to process ultrasound measurements of a patient, wherein the ultrasound system comprises an ultrasound unit operable to perform the ultrasound measurements of the patient, wherein the method comprises:
- obtaining (410) user interaction data of one or more interactions of a current user performing a current task at the ultrasound system, wherein the ultrasound system provides user-facing functionality for performing the current task at multiple different skill levels;
- from the user interaction data, determining (420) a real-time skill estimate of the current user, wherein the real-time skill estimate indicates a current ability of the current user to perform the current task; and
- customizing (430) the ultrasound system for performing the current task at a skill level corresponding to the real-time skill estimate of the current user.

15. A transitory or non-transitory computer-readable medium (500) comprising data (510) representing instructions which, when executed by a processor system, cause the processor system to perform the computer-implemented method according to claim 14.
